(19) Europäisches Patentamt
European Patent Office
Office européen des brevets

(11) **EP 2 620 507 A1**

(12) **EUROPEAN PATENT APPLICATION**

(43) Date of publication:
**31.07.2013 Bulletin 2013/31**

(51) Int Cl.:
*C12Q 1/54* (2006.01)          *C12M 1/34* (2006.01)
*G01N 33/66* (2006.01)

(21) Application number: **12000479.1**

(22) Date of filing: **25.01.2012**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(71) Applicants:
• **Roche Diagnostics GmbH**
  **68305 Mannheim (DE)**
  Designated Contracting States:
  **DE**
• **F. Hoffmann-La Roche AG**
  **4070 Basel (CH)**
  Designated Contracting States:
  **AL AT BE BG CH CY CZ DK EE ES FI FR GB GR HR HU IE IS IT LI LT LU LV MC MK MT NL NO PL PT RO RS SE SI SK SM TR**

(72) Inventor: **The designation of the inventor has not yet been filed**

(74) Representative: **Riwotzki, Karsten**
**Roche Diagnostics GmbH**
**Patent Department**
**Sandhofer Straße 116**
**68305 Mannheim (DE)**

(54)     **Method for the evaluation of the quality of a test element**

(57)     The present invention relates to a method for evaluating the quality of an analysis element which is useful for determining the concentration of an analyte in a liquid sample, and to an analysis system which implements such a method. A reaction of the sample with a reagent system includes a complex formation of at least an enzyme and a co-enzyme, wherein a chemically modified form of the co-enzyme is a fluorophore. The quantity and/or the luminescence properties of said fluorophore relate to the concentration of the analyte. The quality of the analysis element is evaluated using an evaluation algorithm, wherein the evaluation algorithm comprises obtaining information relating to a measured enzyme activity $A_{Enz}$(measured), wherein said information is at least partly derived from a measurement of the fluorescence lifetime of the fluorophore.

**Fig. 2**

EP 2 620 507 A1

**Description**

**TECHNICAL FIELD OF THE INVENTION**

The present application relates to a method for evaluating the quality of an analysis element which is useful for determining the concentration of an analyte in a liquid sample, and more particularly to the assaying of medical samples such as blood and/or interstitial liquid, for analytes contained therein, such as glucose. Furthermore, the application relates to an analysis system for determining the concentration of an analyte in a liquid sample which implements such a method.

**BACKGROUND**

In methods for determining the concentration of analyte based on a fluorescence measurement, a reagent system is used which reacts with the sample resulting in a change of the amount of a fluorophore. This change of quantity can in principle be both an increase by formation of a fluorophore and also a decrease by consumption of a fluorophore. The reaction system is selected so that the change of the quantity of the fluorophore is characteristic of the desired analytical concentration. The analytical method includes the measurement of a measurement variable which correlates with the quantity of the fluorophore. The concentration is determined on the basis of the resulting measurement values by means of an evaluation algorithm.

Methods of this type are generally known. Reference can be made to the following documents, for example EP 0 293 732 A2, US 2005/0214891 A1, and US 2006/0003397 A1. All of these publications describe a similar test protocol, in which the reagent system contains the enzyme-coenzyme pair glucose dehydrogenase (GlucDH)/nicotinamide-adenine dinucleotide (NAD). Upon the reaction with this reagent system, under the influence of the GlucDH, a hydride ion is cleaved from the glucose and transferred to the NAD, so that NADH forms. The resulting quantity of NADH is characteristic of the glucose concentration. NADH is a strong fluorophore, whose concentration can be determined by a measurement of the fluorescence intensity. The correlation of the measured fluorescence intensity to the analyte concentration is typically determined by calibration. NADH is also a dye. Therefore, it can be favorable to additionally perform a photometric measurement (for example, measurement of the optical absorption at a specific wavelength).

This type of method has basically been known for some time. For example, reference is made in US 2005/0214891 A1 to a publication of Narayanaswamy et al. from the year 1988, in which a fluorescence measurement using GlucDH and NAD was employed for glucose determination. However, an array of fundamental problems is connected to the measurement of the fluorescence intensity, which are discussed, for example, in the book by J. R. Lakovicz, "Principles of Fluorescence Spectroscopy" (Springer Science and Business Media, 2006), and include the following:

○ The intensity of the detected fluorescence signal is a function of a plurality of instrument-specific factors, such as the power of the light source, the transmission of the optical components in the light path, or the sensitivity of the detector. Uncontrolled changes of these factors impair the measurement precision.
○ A further error source in intensity measurements results from nonspecific light, which reaches the detector from the environment and can cause an uncontrolled signal change.
○ The intensity of the measured fluorescence light is not only a function of the quantity of the fluorophore. Rather it is also significantly influenced by its molecular environment in the sample. In particular processes which are summarized under the term fluorescence quenching contribute thereto in.
○ The position and orientation of the molecule can change between absorption and emission because in the statistical mean a time in the order of nanoseconds passes between the excitation of a molecule and the emission of a light quantum. Interfering influences result therefrom in regard to the fluorescence intensity, in particular temperature dependence.
○ The fluorescence is generally excited by ultraviolet light. Photochemical reactions of the electronically excited state may cause bleaching of the fluorophore. This is a further error source.

These problems may be reduced by calibration of the measurement of the fluorescence intensity using a fluorophore whose fluorescence intensity is known. However, this is very complex and is only suitable for sophisticated laboratory measurements.

WO 94/00602 A proposes an in vivo method for detecting an analyte in an individual, using a sensor comprising a fluorescent reagent for detecting said analyte, wherein the fluorescence may be measured e.g. by measuring changes in the excited state lifetime of said fluorophore.

EP 2 022 859 A proposes an improved measurement method wherein fluorescence lifetime measurements of a fluorophore are used in the determination of analyte concentration. Said improved method takes into account the aforementioned measurement errors and interferences, and is suitable to provide more reliable test results.

However, the state of the art in this context does not address the aspect of detecting and/or correcting irregular test results which arise from a degradation of the reagent test system used. Such degradation may occur in particular due to the effects of long storage times, including detrimental environmental conditions, such as high temperatures or high humidity. In these cases, the performance of the reagent test system used may be affected, indicating a result of an analyte level which is too low. This can be particularly critical because a test result may be displayed to the user without the error resulting from the poor performance of the reagent test system being detected.

On this basis, it is an object of the present invention to propose a method which allows with little expense an improved measurement precision, in particular in regard to the described degradation of the reagent test system. Moreover, the method should be suitable for small devices which operate as simply and cost-effectively as possible.

## SUMMARY

This object and others that will be appreciated by a person of ordinary skill in the art have been achieved according to the embodiments of the present invention disclosed herein.

In a first aspect, the present invention comprises a method for evaluating the quality of an analysis element which is useful for determining the concentration of an analyte in a liquid sample, comprising:

(a) providing an analysis element in which a reagent system is operatively integrated, wherein the reagent system contains at least one enzyme and at least one co-enzyme, wherein a reaction of the sample with the reagent system includes a complex formation of at least the enzyme and the co-enzyme, and wherein a chemically modified form of the co-enzyme is a fluorophore;

(b) reacting the sample with the reagent system in order to produce a change of the quantity and/or of the luminescence properties of the fluorophore, wherein the quantity and/or the luminescence properties of the fluorophore relate to the concentration of the analyte;

(c) optionally measuring a first measurement variable,

(c') measuring at least a second measurement variable, wherein the first and the second measurement variable are characteristic for the quantity of the fluorophore and/or for the degree of complexation of the fluorophore, and wherein the second measurement variable is the fluorescence lifetime of the fluorophore;

(d) optionally determining a first concentration $c1_{initial}$ of the fluorophore and/or of the analyte on the basis of the first measurement variable, using a pre-set enzyme activity $A_{Enz}(preset)$;

(e) determining a second concentration $c2_{ref}$ of the fluorophore and/or of the analyte on the basis of the second measurement variable, using the pre-set enzyme activity $A_{Enz}(preset)$; and/or determining the measured enzyme activity $A_{Enz}(measured)$ on the basis of the second measurement variable and taking into account either the first concentration $c1_{initial}$ from step (d) or a known concentration $c1_{known}$ of the analyte in the sample; and

(f) evaluating the quality of the analysis element using an evaluation algorithm, wherein the evaluation algorithm comprises obtaining information relating to the measured enzyme activity $A_{Enz}(measured)$, wherein said information is at least partly derived from the measurement of the fluorescence lifetime of the fluorophore, either directly by determining the measured enzyme activity $A_{Enz}(measured)$ according to step (e), or indirectly by comparing the second concentration $c2_{ref}$ to the first concentration $c1_{initial}$.

In another aspect, the invention refers to an analysis system for determining the concentration of an analyte in a liquid sample which is adapted to perform the method.

It has been established in EP 2 022 859 A that in analysis systems in which the reagents of the reagent system are integrated in dry form in an analysis element, the fluorescence lifetime is surprisingly dependent to such a high degree on the analyte concentration that measurement values of this measurement variable may advantageously be used in the algorithm for determining the analytical result (desired concentration). This may be explained as follows for the case of a glucose test using the reagent system GlucDH/NAD.

The NADH formed as a result of the reaction of the glucose with the enzyme-coenzyme pair GlucDH/NAD forms a complex with the GlucDH. This complex formation influences the fluorescence lifetime of the NADH: the fluorescence of the free NADH is short-lived, because molecules in the surrounding liquid act as a quencher. In the complex, the NADH molecule is largely protected from the effect of quenchers and the fluorescence is significantly longer-lived.

These facts are known from the literature. At the time of filing EP 2 022 859 A, however, it had been completely unexpected that this effect is so pronounced under the conditions prevailing in practical tests that the changes of the analyte concentration in blood samples occurring in practice result in a well-measurable shift of the mean fluorescence lifetime. On the basis of statements in the literature, it would have been expected that the degree of binding of the complex NADH/GlucDH is very high. The fluorescent light emitted by the complex NADH/GlucDH has a very much higher intensity than the fluorescence of free NADH. Therefore, it would be expected that the observed fluorescence lifetime depends only minimally from the free NADH and the lifetime effect caused by concentration changes in the physiological range is immeasurably low. However, it was established in the context of EP 2 022 859 A that under practical conditions a very high proportion of the NADH (over 90%) is not complexed.

According to the invention, optionally a first and at least a second measurement variable which are characteristic for the quantity of the fluorophore and/or for the degree of complexation of the fluorophore are measured, wherein the second measurement variable is the fluorescence lifetime of the fluorophore. Herein, the term "degree of complexation" refers to the amount of fluorophore being attached to the enzyme in the enzyme/co-enzyme complex (i.e. being complexed), as opposed to the fluorophore being present in its free form (i.e. without being attached to the enzyme).

The fluorescence lifetime provides a second, independent source of information about the fluorescence of the reaction products, in addition to the fluorescence intensity. It can be used independently (i.e., without other information) for determining the desired analyte concentration. However, it is preferably used in combination with the measurement of the fluorescence intensity. This does not require any additional expense for measurement technology, because typical methods for measuring the fluorescence lifetime simultaneously provide measurement results about the fluorescence intensity.

In order to use the fluorescence lifetime for the determination of the desired analyte concentration, it can be expedient to measure it and to introduce the measurement values resulting from the measurement into the evaluation algorithm in any suitable way. However, such a measurement (in the strict meaning, i.e., measurement of previously unknown measurement values) is not mandatory. Rather, the fluorescence lifetime can also be used in a way in which no measurement in the meaning that (previously unknown) measurement values of the fluorescence lifetime are determined, is required. One possibility is to measure the fluorescence intensity within a defined time window, the time window being adapted to the (known) fluorescence lifetime of a fluorophore resulting from the reaction of the sample with the reagent system. The measurement value resulting from such a measurement is related to the fluorescence lifetime. Hereafter it is also designated "lifetime-related fluorescence intensity". It may be used in the determining of the second concentration $c2_{ref}$ of the fluorophore and/or of the analyte on the basis of the second measurement variable. Therefore also in this case, it is correct to say that the fluorescence lifetime is used in the algorithm for determining the analyte concentration.

Due to the fact that a measurement of the fluorescence lifetime (in the explained strict meaning) is not absolutely necessary, the analysis instrument of a corresponding analysis system does not necessarily have to have a measuring unit which is adapted for measuring the fluorescence lifetime of the fluorophore. Rather, the possibility alternatively or additionally exists that the measuring unit of the analysis instrument is implemented so that it considers the fluorescence lifetime in another way, for example, using the discussed time window which is set during the intensity measurement.

Suitable methods for measuring the fluorescence lifetime are known. One means that is, in principle, suitable for the invention is a time-resolved fluorescence detection, in which a very short pulse of excitation light is emitted and the curve of the resulting emission curve is detected using suitable detection methods having high time resolution. However, a phase modulation method operating with continuous emission is preferably used in the context of the invention. More detailed information about suitable methods may be taken from the literature. Reference is to be made in particular to the book of Lakovicz cited above, and to the following further publications, as well as the literature cited therein: T. G. Scott et al., "Emission Properties of NADH. Studies of Fluorescence Lifetimes..." (J. Am. Chem. Soc., 1970, 687-695); U.S. Pat. No. 5,485,530; and EP 0 561 653 A1.

According to the invention, a chemically modified form of the co-enzyme is a fluorophore. Thus, any co-enzyme may be used in the invention which can be transformed into a modified form having fluorescence properties. Co-enzymes which can be transformed into a fluorophore by a rather simple chemical change are particularly useful for the purpose of the invention: This is the case e.g. if the chemical change is an oxidation or a reduction, which may be effected by simple transformation steps such as protonation, de-protonation, or hydride transfer, which transformation steps may advantageously be promoted by enzymatic action. Preferably, the co-enzyme is chemically modified into a fluorophore during reaction of the sample with the reagent system. When NAD or derivatives thereof, such as carbaNAD, carbaNADP and derivatives thereof, are used in the invention, then usually NAD (or the corresponding derivatives thereof, respectively) acts as the co-enzyme, whereas the corresponding reduced form (such as NADH, carbaNADH, etc., i.e. the chemically modified form of the co-enzyme) represents the fluorophore. In the context of the present invention, since the fluorophore is a modified form of the co-enzyme, the term "co-enzyme" may also sometimes be employed when reference is made to the fluorophore (despite the fact that the fluorophore may itself not act as a co-enzyme, as it is the case e.g. for the pairs of co-enzyme and fluorophore comprising NAD/NADH, carbaNAD/carbaNADH, and their corresponding derivatives).

Optionally, the method of the invention makes use of the pre-set enzyme activity $A_{Enz}$(preset), namely in the determining of the first concentration $c1_{initial}$ of the fluorophore and/or of the analyte on the basis of the first measurement variable, and/or in the determining of the second concentration $c2_{ref}$ of the fluorophore and/or of the analyte on the basis of the second measurement variable. It is to be noted that said pre-set enzyme activity $A_{Enz}$(preset) does not need to be actually measured for carrying out the method of the invention. Rather, it may be sufficient to ensure that there is an amount of enzyme present in the reagent system which provides for an overall enzyme activity which is, under usual measurement conditions, large enough such that the reaction of the sample with the reagent system will proceed nearly until a reaction equilibrium is reached. For example, if in the determining of the first concentration $c1_{initial}$, the first measurement variable is absorption of the fluorophore, such as by using a reflection measurement, then the actual specific value of $A_{Enz}$(preset) is not needed, but $A_{Enz}$(preset) is implicitly considered (namely, by assuming that $A_{Enz}$ (preset) is sufficiently high such that $c1_{initial}$ can be reasonably determined). Typically, the pre-set enzyme activity $A_{Enz}$ (preset), or at least its order of magnitude, can be derived or estimated from the amount of enzyme as employed in the reagent test system.

The evaluation algorithm may comprise determining if the enzyme activity $A_{Enz}$(measured) is below a predetermined threshold T1. Said threshold T1 e.g. may be an absolute minimum value of enzyme activity $A_{Enz}$(min) which is known to be necessary in order to carry out a reliable analyte measurement using the reagent system of the invention. As another advantageous option, which may be used additionally or alternatively, the evaluation algorithm may comprise determining if the difference between the enzyme activity $A_{Enz}$(measured) and the pre-set enzyme activity $A_{Enz}$(preset) is above a predetermined threshold T2.

Additionally or alternatively, the evaluation algorithm comprises determining if the difference between the first concentration $c1_{initial}$ from step (d) and the second concentration $c2_{ref}$ from step (e) is above a predetermined threshold T3.

In one embodiment of the present invention, the analysis element is rejected if the enzyme activity $A_{Enz}$(measured) is below the predetermined threshold T1. Preferably, the analysis element is rejected if the difference between the enzyme activity $A_{Enz}$(measured) and the pre-set enzyme activity $A_{Enz}$(preset) is above the predetermined threshold T2. Additionally or alternatively, it is as well preferred that the analysis element is rejected if the difference between the first concentration $c1_{initial}$ from step (d) and the second concentration $c2_{ref}$ from step (e) is above the predetermined threshold T3.

In this context, it is worth noting that the method of the invention may advantageously be employed by using a control solution (i.e. the sample) of known analyte concentration $c1_{known}$, e.g. a solution containing a known amount of glucose, thus avoiding any need to determine the first and the second concentration $c1_{initial}$ and $c2_{ref}$, respectively. According to step (e), the measured enzyme activity $A_{Enz}$(measured) may be determined on the basis of the second measurement variable (i.e. from the fluorescence lifetime measurement) and taking into account the known concentration $c1_{known}$ of the analyte in the control solution. The measured enzyme activity $A_{Enz}$(measured) may then be used to evaluate the quality of the test element as described herein before. As a result, it may be concluded that a plurality of test elements, e.g. the test strips from an open package or container, should not be used any more.

In another preferred embodiment of the present invention, the information about the enzyme activity $A_{Enz}$ (measured) is used to determine a corrected first concentration $c1_{corr}$ of the fluorophore and/or of the analyte.

Thus, according to a preferred embodiment, the first concentration $c1_{initial}$ of the fluorophore and/or of the analyte is determined according to step (d) on the basis of the first measurement variable, using a pre-set enzyme activity $A_{Enz}$(preset), and the measured enzyme activity $A_{Enz}$(measured) is determined according to step (e) on the basis of the second measurement variable and using the first concentration $c1_{initial}$ from step (d). Then it is determined if $A_{Enz}$ (measured) is below a predetermined threshold T1, and/or if the difference between $A_{Enz}$(measured) and the pre-set enzyme activity $A_{Enz}$(preset) is above a predetermined threshold T2. If one or both of these conditions are fulfilled, then it can be concluded that the enzyme activity actually present in the reagent test system (corresponding to $A_{Enz}$(measured)) differs from the pre-set enzyme activity $A_{Enz}$(preset) to such an extent that either the test element should be rejected, or that a corrected first concentration $c1_{corr}$ of the fluorophore and/or of the analyte should be calculated, e.g. on the basis of a different calibration curve for the reagent system, which calibration curve is specifically related to or adapted to a lower enzyme activity.

In a more preferred embodiment, the determining of the corrected first concentration $c1_{corr}$ comprises an iterative algorithm.

Typically, the first measurement variable may be one of absorption and fluorescence intensity, preferably absorption. In the context of the present invention, the term "absorption" shall comprise any measurement arrangement which is suitable to detect at least some degree of optical absorption. In particular, the optical absorption may be measured by reflection (also referred to as remission) and/or transmission measurements.

In another embodiment, the first measurement variable is absorption, and the method further comprises measuring a third measurement variable which is characteristic for the quantity of the fluorophore, wherein the third measurement variable is fluorescence intensity, and wherein the first, the second and the third measurement variable are used in the evaluation algorithm.

Advantageously, the measurement of the fluorescence lifetime is used to determine a lifetime-related value of the fluorescence intensity (LRFI). The measurement of LRFI values is an example of the fact that in the context of the invention a measurement of a fluorescence lifetime (in the strict meaning) is not absolutely necessary. Rather, by using LRFI values, the measurement of the fluorescence lifetime can be efficiently effected via measurements of fluorescence intensities, wherein the measurements of fluorescence intensity are carried out in a specifically adapted time frame.

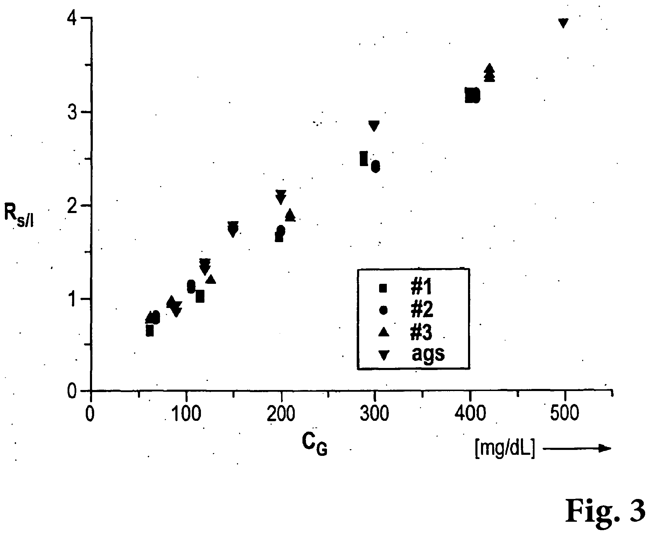

This will be elucidated below in more detail with regard to FIG. 3.

In a preferred embodiment, the fluorescence intensity is measured for at least two different fluorescence lifetimes in order to determine in each case lifetime-related values of the fluorescence intensity.

In a more preferred embodiment, the two lifetime-related values of the fluorescence intensity are related to one another.

Additionally, the measured fluorescence lifetime may be used in a compensation algorithm to compensate for measurement errors which are caused by interfering variables, such as the temperature or humidity conditions, particularly at the time of measurement, or the hematocrit level of the sample.

The fluorophore is preferably selected from the group comprising NADH and NADPH, including their derivatives. In particular, derivatives are suitable in which the molecule part essentially responsible for the fluorescence remains unchanged. In such derivatives, basically similar fluorescence properties are to be expected. Of course, the parameters of the fluorescence, such as the wavelength of the absorption or the emission, may change. Due to the fact that the pyridine ring is essentially responsible for the fluorescence in NAD/NADH, derivatives in which the pyridine ring is not modified are particularly suitable. Derivatives suitable for the present invention are described, for example, in WO 2007/012494 and US 2007/0026476. CarbaNAD, a derivative without glycosyl binding which was already described in 1988, is particularly suitable for use in the method according to the invention (J. T. Slama, Biochemistry 1989, 27, 183 and Biochemistry 1989, 28, 7688). The ribose is substituted therein by a carbocyclic sugar unit. Of course, not all derivatives of NADH and NADPH are equally suitable for the invention. The suitability for use according to the invention can, however, be experimentally examined without problems. Preferably, the fluorophore is NADH or NADPH or a derivative thereof, whose pyridine ring is not modified, in particular carbaNADH or carbaNADPH or a derivative thereof.

Typically, the enzyme may be selected from the group comprising glucose dehydrogenase (E.C.1.1.1.47), lactate dehydrogenase (E.C.1.1.1.27, 1.1.1.28), malate dehydrogenase (E.C. 1.1.1.37), glycerine dehydrogenase (E.C. 1.1.1.6), alcohol dehydrogenase (E.C.1.1.1.1), amino acid dehydrogenase, such as L-amino acid dehydrogenase (E.C. 1.4.1.5), alpha-hydroxybutyrate dehydrogenase, sorbitol dehydrogenase, glucose oxidase (E.C.1.1.3.4), cholesterol oxidase (E.C.1.1.3.6), amino transferases, such as aspartate or alanine amino transferase, 5'-nucleotidase, creatin kinase, glucose 6-phosphate dehydrogenase (E.C.1.1.1.49), NAD dependent cholesterol dehydrogenase (E.C.1.1.1.62), and FAD dependent glucose dehydrogenase (E.C. 1. 1.99. 10). Preferably, the enzyme is selected from the group consisting of glucose dehydrogenase (E.C.1.1.1.47), alcohol dehydrogenase (E.C.1.1.1.1), and glucose 6-phosphate dehydrogenase (E.C.1.1.1.49); more preferably, the enzyme is one of glucose dehydrogenase (E.C.1.1.1.47) and glucose 6-phosphate dehydrogenase (E.C.1.1.1.49). Most preferably, the enzyme is glucose dehydrogenase (E.C.1.1.1.47).

Typically, the liquid sample to be analyzed may be a bodily fluid, specifically blood or interstitial fluid. Preferably, the analyte is glucose.

In a further aspect, the present invention relates to an analysis system for determining the concentration of an analyte in a liquid sample, the system comprising

- a reagent system comprising reagents contained in dry form in an analysis element, the reagent system containing at least one enzyme and at least one co-enzyme, a chemically modified form of the co-enzyme being a fluorophore, a reaction of the sample with the reagent system including a complex formation of at least the enzyme and the co-enzyme, the reaction with the sample resulting in a change of the quantity and/or of the luminescence properties of the fluorophore, the quantity and/or the luminescence properties of the fluorophore relating to the concentration of the analyte;

- an evaluation instrument comprising a measuring unit for optionally measuring a first and measuring at least a second measurement variable which both are characteristic for the quantity of the fluorophore and/or for the degree of complexation of the fluorophore, the measuring unit being adapted for measuring the fluorescence lifetime of the fluorophore as the second measurement variable;

- an evaluation unit for determining the concentration of the fluorophore and/or of the analyte, the evaluation unit being adapted to

   - optionally determine a first concentration $c1_{initial}$ of the fluorophore and/or of the analyte on the basis of the first measurement variable, using a pre-set enzyme activity $A_{Enz}$(pre-set);
   - determine a second concentration $c2_{ref}$ of the fluorophore and/or of the analyte on the basis of the second measurement variable, using the pre-set enzyme activity $A_{Enz}$(pre-set); and/or determine the measured enzyme activity $A_{Enz}$(measured) on the basis of the second measurement variable and taking into account either the first concentration $c1_{initial}$ from step (d) or a known concentration $c1_{known}$ of the analyte in the sample; and
   - evaluate the quality of the analysis element using an evaluation algorithm, wherein the evaluation algorithm comprises obtaining information relating to the measured enzyme activity $A_{Enz}$ (measured), wherein said information is at least partly derived from the measurement of the fluorescence lifetime of the fluorophore, either directly by determining the measured enzyme activity $A_{Enz}$(measured), or indirectly by comparing the second concentration $c2_{ref}$ to the first concentration $c1_{initial}$.

The most prominent advantage of the present invention is that a degradation of the reagent test system can be detected and/or can be corrected for. Inter alia, the following additional advantages may be achieved by the present invention:

  ◯ The problems discussed above are largely avoided. In particular, a calibration of the fluorescence intensity, using a standard fluorophore, is not necessary.
  ◯ The high-frequency measurement results in a reduction of the influence of interfering ambient light.
  ◯ The invention allows increased precision, in particular by elimination or reduction of error sources.

The invention is to be explained in more detail by the following figures and examples.

## BRIEF DESCRIPTION OF THE DRAWINGS

The following detailed description of the embodiments of the present invention can be best understood when read in conjunction with the following drawings, where like structure is indicated with like reference numerals and in which:

  FIG. 1 shows a schematic sketch of the functional components of an analysis system suitable for embodiments of the present invention.
  FIG. 2 shows measurement results in regard to the mean lifetime of the fluorescence as a function of the ratio of the concentrations of GlucDH and NADH for various temperatures.
  FIG. 3 shows a measurement result in regard to the functional relationship between the glucose concentration and the intensity ratio between short-lived and long-lived fluorescence.
  FIG. 4 shows a measurement result of the average fluorescence lifetime versus glucose concentration measured on test strips.
  FIG. 5 shows a measurement result of the average fluorescence lifetime versus enzyme activity.
  FIG. 6 shows an example of a preferred embodiment of the present invention schematically illustrating an iterative evaluation algorithm for correcting the initially determined concentration $c1_{initial}$ by taking into account information about the enzyme activity $A_{Enz}$.

In order that the present invention may be more readily understood, reference is made to the following detailed descriptions and examples, which are intended to illustrate the present invention, but not limit the scope thereof.

## DETAILED DESCRIPTION OF EMBODIMENTS OF THE PRESENT INVENTION

The following descriptions of the embodiments are merely exemplary in nature and are in no way intended to limit the present invention or its application or uses.

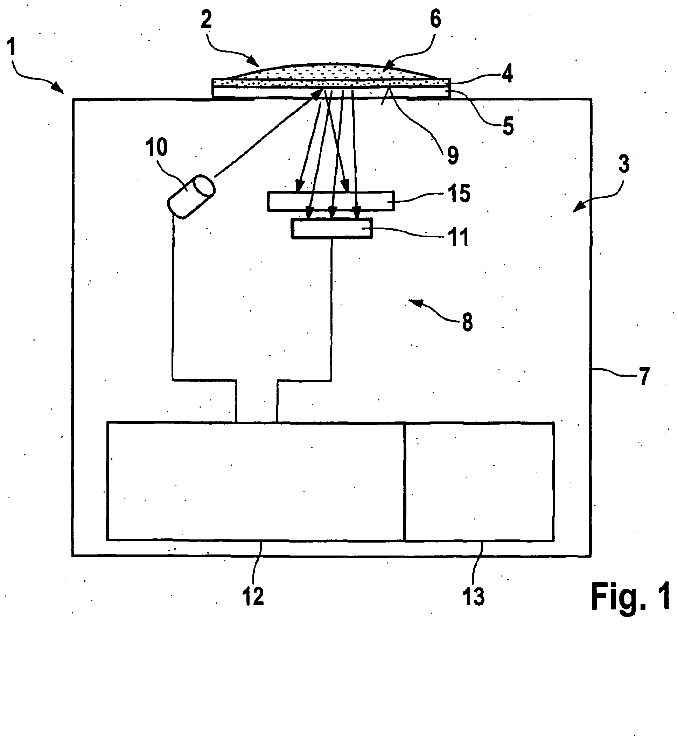

The analysis system shown in very schematic form in FIG. 1 comprises two components adapted to one another, namely an analysis element **2** and an analysis instrument **3**. The analysis element **2** comprises a reagent layer **4,** which contains a reagent system in a suitable matrix, such as a gel matrix. The reagent layer **4** is located on an optically transparent carrier **5**. The reagents of the reaction system are embedded in the matrix of the reagent layer **4** in dry form. When a sample liquid **6** is dispensed onto the free surface of the reagent layer **4** and penetrates therein, the reagents of the reagent system are dissolved and a reaction of the sample liquid occurs, which results in a change of the quantity of a fluorophore, which is a component of the reagent system. Further information about such analysis elements is provided, for example, in the documents U.S. Pat. No. 3,802,842; U.S. Pat. No. 4,061,468; and US 2006/0003397.

The housing **7** of the analysis instrument **3** contains a measuring unit, designated identified as a whole by **8.** It is adapted for determining a measurement variable which is characteristic for the quantity of the fluorophore. The measuring unit **8** includes an excitation light source **10,** such as a laser or an LED, whose light is directed from the back side (through the optically transparent carrier **5**) onto a surface of the reagent layer **4** which faces away from the sample dispensing side and toward the optically transparent carrier **5**. Secondary light is emitted from the analysis zone **9** toward a detector **11** and is detected thereby. The resulting measuring signal is supplied to a signal processing unit **12** and amplified, processed, and digitized in a usual manner. The resulting digitized measurement values are supplied to an analysis unit **13** which contains the required software and hardware to determine the desired concentration of the analyte from the digitized measurement values.

The measuring unit **8** is adapted for detecting fluorescent light emitted from the analysis zone **9.** For this purpose, the access of the primary light originating from the excitation light source **10** is blocked, using typical means such as a filter element **15** shown in FIG. 1, in such a manner that the fluorescent light, which is emitted at a longer wavelength, can be detected selectively by the detector **11.**

So far the design of the analysis system according to the invention is conventionally and therefore does not have to be explained in greater detail. More detailed information is available, for example, from the cited documents of the prior art.

A special feature of the analysis system according to the invention shown in FIG. 1 is that the measuring unit **8** is capable of measuring the fluorescence lifetime and/or to take it into account (in the context of the measurement of another measurement variable). As already noted, the fluorescence lifetime measurement is preferably performed using a phase modulation method. The light emitted from the excitation light source **10** is modulated at high frequency and the desired information about the fluorescence lifetime is determined from a phase shift which is measured by means of the detector **11** and the signal processing unit **12.** Again a more detailed explanation in this regard is not necessary, because supplementary information is available from the literature, in particular the above-mentioned documents.

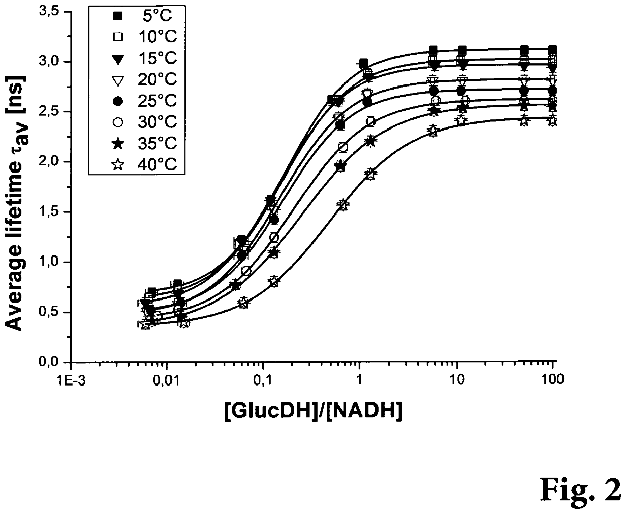

FIG. 2 shows experimental results from the research work which contributed to the invention. The mean fluorescence lifetime $\tau_{av}$ is shown for various concentration ratios of a liquid mixture of GlucDH and NADH. A 23$\mu$M NADH solution having varying concentrations of GlucDH (11.5$\mu$M to 2.3mM) was used to prepare the concentrations specified on the abscissa. The measurements were carried out keeping the optical density smaller than 0.2. The fluorescence lifetime of protein-bound NADH was measured at different temperatures in the range of 5°C to 40°C by reducing NAD to NADH by Glucose, catalyzed by GlucDH. The fluorescence decay was then fitted using a three exponential model, whereby the two lifetimes of free NADH were kept constant.

FIG. 2 shows that the shift of the ratio between free and complexed NADH, which is caused by the variation of the concentration of GlucDH, can be observed well by lifetime measurements, the measurement curve approaching the limiting values $\tau_1$=2.98 ns and $\tau_2$=0.42 ns for very high and very low GlucDH concentrations. The fluorescence lifetime is a measurement variable which is characteristic of the quantity of the fluorophore, as shown by the fact that the degree of complex formation is a function of the amount of the fluorescent species.

As illustrated in Fig. 2, the change in the average fluorescence lifetime is largest for a concentration ratio of enzyme to coenzyme in-between 0.1 and 1. Therefore, it may be advantageous to adapt the concentration of GlucDH in future test elements in a way that the concentration ratio of enzyme to coenzyme is within this range.

Additionally, Fig. 2 shows that the average fluorescence lifetime is also dependent on temperature. While the shape of the curve appears to stay constant, the average lifetimes shift to higher values for lower temperatures. From a practical perspective, the average lifetime may thus be used for a conceivable temperature correction of an analyte sensing system, such as e.g. a glucose monitoring test system.

In the context of the invention it was derived from the measurement results shown in FIG. 2 that the change of the lifetime can be used to get additional information for quantifying an analyte in fluorescence tests, in particular information relating to the enzyme activity as explained herein. These findings can be used analytically in various ways. For example, for the purpose of the invention and analogous to FIG. 2, the mean fluorescence lifetime $\tau_{av}$ may be plotted versus various ratios of the enzyme activity (e.g. of GlucDH) to NADH concentration.

FIG. 3 shows an example of a preferred way of measuring the second measurement variable in order to gain information therefrom for the determination of the first concentration $c2_{ref}$ and/or $A_{Enz}$(measured). Particularly, FIG. 3 illustrates a possibility in which the measurement of the fluorescence lifetime is used for determining a lifetime-related fluorescence intensity (LRFI). Such an LRFI value describes the intensity of the fluorescence as a function of the fluorescence lifetime and thus forms a measure of the relationship of the quantity of a plurality of fluorescent species, which are distinguished by different mean lifetimes, such as complexed NADH in proportion to free NADH. An LRFI value can be determined, for example, on the basis of fluorescence signals, which are excited by short light pulses and are measured at high resolution of time. The decay of the fluorescence signal is a logarithmic function, which is a function of the mean fluorescence lifetimes of the fluorescent species and their concentration. The separate intensities of the two signal components may be determined by mathematical fitting of such measurement curves.

FIG. 3 shows the dependence of such LRFI values on the glucose concentration for a glucose fluorescence test using analysis elements as are described in US 2005/0214891 A1 and US 2006/0003397 A1. A ratio $R_{s/l}$ between the LRFI value for short-lived free NADH and the LRFI value for long-lived complexed NADH is shown. The measurements were performed using blood samples of different hematocrit values and using an aqueous glucose solution. The resulting measurement values are identified by different symbols in FIG. 3. Symbols for #1 through #3 indicate the numbers of samples having different hematocrit concentrations (very high-normal range-very low); "ags" means "aqueous glucose solution". It was shown that the correlation between the glucose concentration and the $R_{s/l}$ value is very good, the measured values being largely independent of the hematocrit value, i.e., of the concentration of the blood cells in the sample.

In FIG. 3, two LRFI values are related to one another. $R_{s/l}$ was calculated according to

$$R_{s/l} \quad = \quad \frac{A_2 \cdot \tau_2}{A_1 \cdot \tau_1}$$

Therein A designates the amplitude, i.e., a measure of the intensity, and $\tau$ designates the fluorescence lifetime of the two fluorescent species having different lifetimes. The value $R_{s/l}$ is calibrated against the analyte concentration (or, in a first step, against the concentration of the fluorophore) and then used in an algorithm as a measure for the analyte (or the fluorophore) concentration.

There are numerous other possibilities for relating LRFI values to one another so that a computed variable is formed, which can be used, via a calibration, as a measure for the desired analyte (or fluorophore) concentration. For example, a mean fluorescence lifetime (referred to as $\tau_m$ here) can be calculated according to

$$\tau_m = \frac{A_1\tau_1 + A_2\tau_2}{A_1 + A_2}$$

It is particularly preferred to perform an area-weighted averaging according to

$$\tau_m \quad = \quad \frac{A_1\tau_1{}^2 + A_2\tau_2{}^2}{A_1\tau_1 + A_2\tau_2}$$

All of these examples share the feature that the fluorescence lifetimes and the fluorescence intensities of two fluorescent species are used as characteristic measurement variables for the analysis. They are related to one another according to a predefined mathematical equation in order to determine a computed variable, which can be calibrated against the desired analyte (or fluorophore) concentration. The use of two simultaneously measured values and the calculation of a quotient has the result that measurement errors which influence both fluorescence measurement values in the same way, for example, due to contaminants or small flaws in the optical measuring channel, cause no or little deterioration of the measurement result, less than in previously known measurements of the fluorescence intensity.

The measurement of LRFI values is an example of the fact that in the context of the invention, a measurement of a fluorescence lifetime (in the strict meaning) is not absolutely necessary. As described above, the measuring unit of the analysis instrument can also be implemented so that it detects a fluorescence intensity of a predefined time window and an LRFI value is thus measured. Preferably two or more LRFI values of this type are related to one another in order to determine a computed variable which can be calibrated against the analyte concentration, similarly as explained in the above examples.

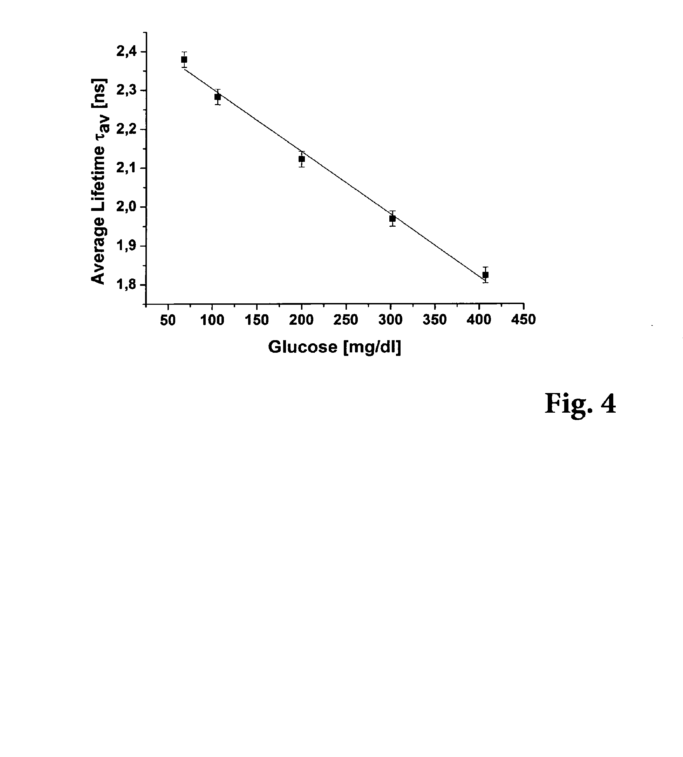

Figure 4 shows the average fluorescence lifetime $\tau_{av}$ versus the glucose concentration, as measured on test strips using a confocal microscope (Axiovert 100, Zeiss, Germany) equipped with a water immersion objective (NA=1,2, Plan Apo, Olympus, Japan). The test strips were excited with a pulsed laser diode (LD) at 375nm. Blood with varying concentrations of glucose was applied on several test strips, containing NAD and GlucDH. The average fluorescence lifetime $\tau_{av}$ exhibits a linear behavior with glucose concentration.

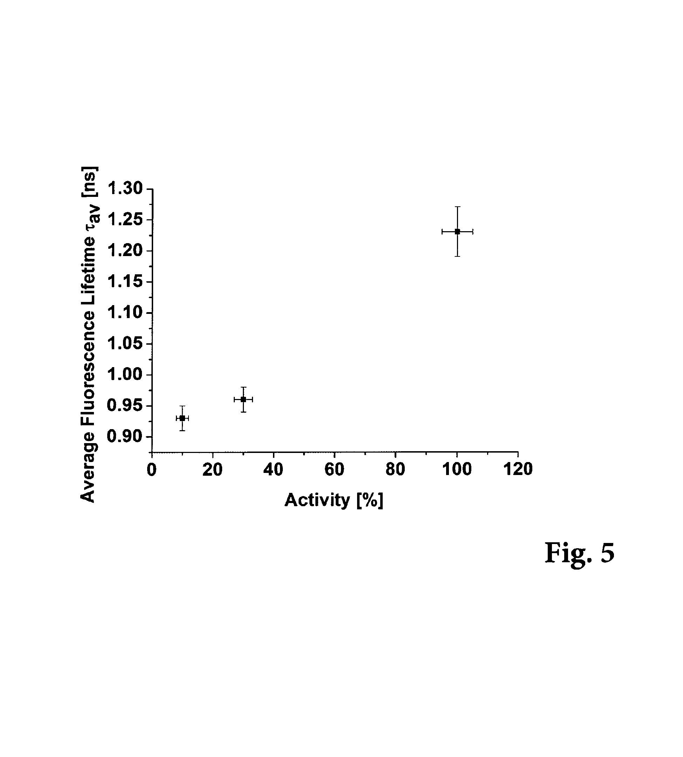

FIG. 5 shows the average fluorescence lifetime $\tau_{av}$ versus enzyme activity. These experiments were carried out using fluorescence lifetime imaging measurements on glass substrates to which the reagents had been applied, wherein the fluorescence lifetime was monitored in two time frames, namely 0.5-1 ns, and 3-3.5 ns). The average fluorescence lifetime $\tau_{av}$ decreases with enzyme activity. This can be explained as follows: The less active the enzyme is, the more molecules of NADH are present in their free form, i.e. not attached to the enzyme, and the average fluorescence lifetime $\tau_{av}$ will gradually point to the fluorescence lifetime of free NADH. In this way, the dependence of the fluorescence lifetime of the fluorophore from the (actual) enzyme activity could be shown.

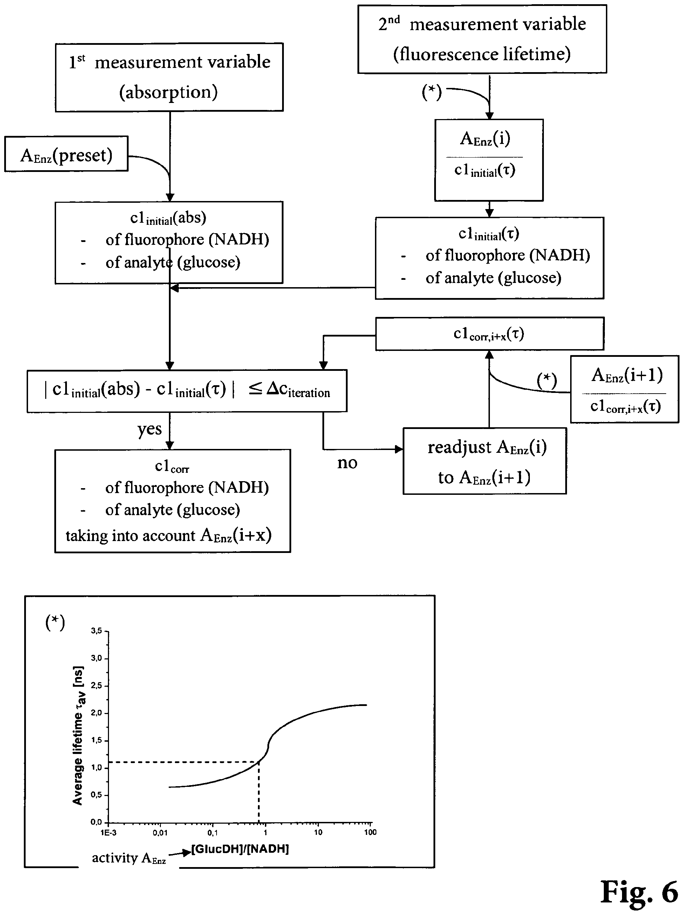

FIG. 6 shows an example of a preferred embodiment of the present invention schematically illustrating an iterative evaluation algorithm for correcting the initially determined concentration $c1_{initial}$ by taking into account information about the enzyme activity $A_{Enz}$. This example assumes a system wherein as a first measurement variable the absorption of the fluorophore NADH is measured, and as a second measurement variable the fluorescence lifetime of NADH is measured, according to step (c) and (c'), respectively. From the absorption measurement, a first concentration $c1_{initial}$(abs) of NADH (or of the analyte, i.e. glucose) can be determined using the first measurement variable and a pre-set enzyme activity $A_{Enz}$(preset). From the measured fluorescence lifetime of NADH, information is obtained relating to the ratio of an enzyme activity $A_{Enz}(i)$ (which e.g. may correspond to the enzyme activity $A_{Enz}$(preset)) to a concentration $c1_{initial}(\tau)$ of NADH. Since the concentration $c1_{initial}(\tau)$ of NADH depends on the two variables (a) measured fluorescence lifetime of NADH, and (b) the actual enzyme activity $A_{Enz}$(actual), and since the fluorescence lifetime of NADH is known at this stage, $c1_{initial}(\tau)$ of NADH may be calculated from the measured fluorescence lifetime of NADH by using the enzyme activity $A_{Enz}(i)$ as an initial enzyme activity to begin an iteration process. Next, the difference between the first concentration $c1_{initial}$(abs) of NADH from the absorption measurement is compared to the calculated concentration $c1_{initial}(\tau)$ of NADH from the fluorescence lifetime measurement. If said difference (i.e. the absolute value thereof) is above a pre-set threshold determining the iteration process (e.g. above 1 mg/dL), then the enzyme activity $A_{Enz}(i)$ should be adjusted to a more suitable value $A_{Enz}(i+l)$. Using the modified enzyme activity $A_{Enz}(i+1)$, a corrected concentration $c1_{corr}(\tau)$ may be calculated. The calculated corrected concentration $c1_{corr}(\tau)$ of NADH (being based on the fluorescence lifetime measurement) may again be compared to the first concentration $c1_{initial}$(abs) of NADH (being based on the absorption measurement). This iteration process may proceed until the iteration criterion is fulfilled, e.g. until the difference between $c1_{initial}$(abs) of NADH and $c1_{corr,i+x}(\tau)$ of NADH is below a pre-set threshold, thereby obtaining an adjusted enzyme activity $A_{Enz}(i+x)$, with x being an integer. Taking the adjusted enzyme activity $A_{Enz}(i+x)$ into account, a corrected concentration $c1_{corr}$(abs) of NADH, and hence a corrected concentration of the analyte of interest, may be calculated, e.g. by using a different calibration curve which is more appropriate for a lower enzyme activity, and may be displayed to the user.

In the context of the present invention, it is to be understood that the term "determining a concentration" may include the case where said concentration value itself actually is not calculated, but where the signal intensity obtained from the measurement is employed. As the skilled person will readily appreciate, this approach is feasible because the measured signal intensity is related to any concentration value derived therefrom. In particular, this applies to determining a first concentration $c1_{initial}$ of the fluorophore and/or of the analyte on the basis of the first measurement variable, and to determining a second concentration $c2_{ref}$ of the fluorophore and/or of the analyte on the basis of the second measurement variable.

For the purpose of the invention, not only the described system, in which glucose is determined with the aid of GlucDH and NAD, may be used. Rather, very generally cases are suitable in which a fluorophore is used which fluoresces in at least two species having different fluorescence lifetimes. In particular, according to the invention the reaction of the sample with the reagent system includes complex formation with participation of at least two molecules, namely an enzyme/coenzyme pair, wherein a chemically modified form of the co-enzyme is a fluorophore.

The features disclosed in the above description, the claims and the drawings may be important both individually and in any combination with one another for implementing the invention in its various embodiments.

It is noted that terms like "preferably", "commonly", and "typically" are not utilized herein to limit the scope of the claimed invention or to imply that certain features are critical, essential, or even important to the structure or function of the claimed invention. Rather, these terms are merely intended to highlight alternative or additional features that may or may not be utilized in a particular embodiment of the present invention.

For the purposes of describing and defining the present invention it is noted that the term "substantially" is utilized herein to represent the inherent degree of uncertainty that may be attributed to any quantitative comparison, value, measurement, or other representation. The term "substantially" is also utilized herein to represent the degree by which a quantitative representation may vary from a stated reference without resulting in a change in the basic function of the subject matter at issue.

Having described the present invention in detail and by reference to specific embodiments thereof, it will be apparent that modifications and variations are possible without departing from the scope of the present invention defined in the claims. More specifically, although some aspects of the present invention are identified herein as preferred or particularly advantageous, it is contemplated that the present invention is not necessarily limited to these preferred aspects of the present invention.

**Claims**

1. A method for evaluating the quality of an analysis element which is useful for determining the concentration of an analyte in a liquid sample, comprising:

    (a) providing an analysis element in which a reagent system is operatively integrated, wherein the reagent system contains at least one enzyme and at least one co-enzyme, wherein a reaction of the sample with the reagent system includes a complex formation of at least the enzyme and the co-enzyme, and

wherein a chemically modified form of the co-enzyme is a fluorophore;

(b) reacting the sample with the reagent system in order to produce a change of the quantity and/or of the luminescence properties of the fluorophore, wherein the quantity and/or the luminescence properties of the fluorophore relate to the concentration of the analyte;

(c) optionally measuring a first measurement variable;

(c') measuring at least a second measurement variable,

wherein the first and the second measurement variable are characteristic for the quantity of the fluorophore and/or for the degree of complexation of the fluorophore, and wherein the second measurement variable is the fluorescence lifetime of the fluorophore;

(d) optionally determining a first concentration $c1_{initial}$ of the fluorophore and/or of the analyte on the basis of the first measurement variable, using a pre-set enzyme activity $A_{Enz}(preset)$;

(e) determining a second concentration $c2_{ref}$ of the fluorophore and/or of the analyte on the basis of the second measurement variable, using the pre-set enzyme activity $A_{Enz}(preset)$; and/or determining the measured enzyme activity $A_{Enz}(measured)$ on the basis of the second measurement variable and taking into account either the first concentration $c1_{initial}$ from step (d) or a known concentration $c1_{known}$ of the analyte in the sample; and

(f) evaluating the quality of the analysis element using an evaluation algorithm, wherein the evaluation algorithm comprises obtaining information relating to the measured enzyme activity $A_{Enz}(measured)$, wherein said information is at least partly derived from the measurement of the fluorescence lifetime of the fluorophore, either directly by determining the measured enzyme activity $A_{Enz}(measured)$ according to step (e), or indirectly by comparing the second concentration $c2_{ref}$ to the first concentration $c1_{initial}$.

**2.** The method according to claim 1, wherein the evaluation algorithm comprises determining if the enzyme activity $A_{Enz}(measured)$ is below a predetermined threshold T1, and/or determining if the difference between the enzyme activity $A_{Enz}(measured)$ and the pre-set enzyme activity $A_{Enz}(preset)$ is above a predetermined threshold T2.

**3.** The method according to claim 1 or 2, wherein the evaluation algorithm comprises determining if the difference between the first concentration $c1_{initial}$ and the second concentration $c2_{ref}$ from step (e) is above a predetermined threshold T3.

**4.** The method according to claim 2 or 3, wherein the analysis element is rejected if the enzyme activity $A_{Enz}(measured)$ is below the predetermined threshold T1, and/or if the difference between the enzyme activity $A_{Enz}(measured)$ and the pre-set enzyme activity $A_{Enz}(pre-set)$ is above the predetermined threshold T2, and/or if the difference between the first concentration $c1_{initial}$ from step (d) and the second concentration $c2_{ref}$ from step (e) is above the predetermined threshold T3.

**5.** The method according to any of the preceding claims, wherein the information about the enzyme activity $A_{Enz}(measured)$ is used to determine a corrected first concentration $c1_{corr}$ of the fluorophore and/or of the analyte.

**6.** The method according to claim 5, wherein the determining of the corrected first concentration $c1_{corr}$ comprises an iterative algorithm.

**7.** The method according to any of the preceding claims, wherein the first measurement variable is one of absorption and fluorescence intensity.

**8.** The method according to claim 7, wherein the first measurement variable is absorption, and wherein the method further comprises measuring a third measurement variable which is characteristic for the quantity of the fluorophore, wherein the third measurement variable is fluorescence intensity, and wherein the first, the second and the third measurement variable are used in the evaluation algorithm.

**9.** The method according to any of the preceding claims, wherein the measurement of the fluorescence lifetime is used to determine a lifetime-related value of the fluorescence intensity.

**10.** The method according to claim 9, wherein the fluorescence intensity is measured for at least two different fluorescence lifetimes in order to determine in each case lifetime-related values of the fluorescence intensity.

**11.** The method according to claim 10, wherein the two lifetime-related values of the fluorescence intensity are related to one another.

12. The method according to any of the preceding claims, wherein the measured fluorescence lifetime is used in a compensation algorithm to compensate for measurement errors which are caused by interfering variables.

13. The method according to any of the preceding claims, wherein the fluorophore is NADH or NADPH or a derivative thereof, whose pyridine ring is not modified, in particular carbaNADH or carbaNADPH or a derivative thereof.

14. The method according to any of the preceding claims, wherein the enzyme is selected from the group consisting of glucose dehydrogenase (E.C.1.1.1.47), lactate dehydrogenase (E.C.1.1.1.27, 1.1.1.28), malate dehydrogenase (E.C.1.1.1.37), glycerine dehydrogenase (E.C.1.1.1.6), alcohol dehydrogenase (E.C.1.1.1.1), amino acid dehydrogenase, such as L-amino acid dehydrogenase (E.C.1.4.1.5), alpha-hydroxybutyrate dehydrogenase, sorbitol dehydrogenase, glucose oxidase (E.C.1.1.3.4), cholesterol oxidase (E.C.1.1.3.6), amino transferases, such as aspartate or alanine amino transferase, 5'-nucleotidase, creatin kinase, glucose 6-phosphate dehydrogenase (E.C.1.1.1.49), NAD dependent cholesterol dehydrogenase (E.C.1.1.1.62), and FAD dependent glucose dehydrogenase (E.C. 1.1.99.10).

15. The method according to any of the preceding claims, wherein the analyte is glucose.

16. An analysis system for determining the concentration of an analyte in a liquid sample, the system comprising

- a reagent system comprising reagents contained in dry form in an analysis element, the reagent system containing at least one enzyme and at least one co-enzyme, a chemically modified form of the co-enzyme being a fluorophore, a reaction of the sample with the reagent system including a complex formation of at least the enzyme and the co-enzyme, the reaction with the sample resulting in a change of the quantity and/or of the luminescence properties of the fluorophore, the quantity and/or the luminescence properties of the fluorophore relating to the concentration of the analyte;
- an evaluation instrument comprising a measuring unit for optionally measuring a first and measuring at least a second measurement variable which both are characteristic for the quantity of the fluorophore and/or for the degree of complexation of the fluorophore, the measuring unit being adapted for measuring the fluorescence lifetime of the fluorophore as the second measurement variable;
- an evaluation unit for determining the concentration of the fluorophore and/or of the analyte, the evaluation unit being adapted to

- optionally determine a first concentration $c1_{initial}$ of the fluorophore and/or of the analyte on the basis of the first measurement variable, using a pre-set enzyme activity $A_{Enz}$(pre-set);
- determine a second concentration $c2_{ref}$ of the fluorophore and/or of the analyte on the basis of the second measurement variable, using the pre-set enzyme activity $A_{Enz}$(pre-set); and/or determine the measured enzyme activity $A_{Enz}$(measured) on the basis of the second measurement variable and taking into account either the first concentration $c1_{initial}$ from step (d) or a known concentration $c1_{known}$ of the analyte in the sample; and
- evaluate the quality of the analysis element using an evaluation algorithm, wherein the evaluation algorithm comprises obtaining information relating to the measured enzyme activity $A_{Enz}$(measured), wherein said information is at least partly derived from the measurement of the fluorescence lifetime of the fluorophore, either directly by determining the measured enzyme activity $A_{Enz}$(measured), or indirectly by comparing the second concentration $c2_{ref}$ to the first concentration $c1_{initial}$.

**Fig. 1**

Fig. 2

Fig. 3

**Fig. 4**

Fig. 5

Fig. 6

Europäisches
Patentamt

European
Patent Office

Office européen
des brevets

# EUROPEAN SEARCH REPORT

Application Number

EP 12 00 0479

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2010/227348 A1 (PETRICH WOLFGANG [DE] ET AL) 9 September 2010 (2010-09-09) * paragraph [0052] - paragraph [0053]; claims; figures; examples * | 1-16 | INV. C12Q1/54 C12M1/34 G01N33/66 |
| A,D | EP 0 293 732 A2 (ABBOTT LAB [US]) 7 December 1988 (1988-12-07) * abstract; claims * | 1-16 | |
| X | WO 2009/015870 A1 (HOFFMANN LA ROCHE & CO AG F) 5 February 2009 (2009-02-05) * abstract; claims; figures * | 1-16 | |
| A | WO 2010/105850 A2 (ROCHE DIAGNOSTICS GMBH [DE]; HOFFMANN LA ROCHE [CH]; HAAR HANS-PETER []) 23 September 2010 (2010-09-23) * abstract; claims * | 1-16 | |
| A | EVANS N D ET AL: "Glucose-dependent changes in NAD(P)H-related fluorescence lifetime of adipocytes and fibroblasts in vitro: Potential for non-invasive glucose sensing in diabetes mellitus", JOURNAL OF PHOTOCHEMISTRY AND PHOTOBIOLOGY B: BIOLOGY, ELSEVIER SCIENCE S.A., BASEL, CH, vol. 80, no. 2, 1 August 2005 (2005-08-01) , pages 122-129, XP027789524, ISSN: 1011-1344 [retrieved on 2005-08-01] * page 124 - page 128 * | 1-16 | TECHNICAL FIELDS SEARCHED (IPC) C12Q C12M G01N |
| A | US 7 857 760 B2 (BRISTER MARK [US] ET AL) 28 December 2010 (2010-12-28) * column 93 - column 95; figure 18a * | 1-16 | |

-/--

The present search report has been drawn up for all claims

| Place of search | Date of completion of the search | Examiner |
|---|---|---|
| Munich | 20 June 2012 | Gonçalves Mauger, M |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

| | Europäisches Patentamt European Patent Office Office européen des brevets | **EUROPEAN SEARCH REPORT** | | **Application Number** EP 12 00 0479 |
|---|---|---|---|---|

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | US 6 055 060 A (BOLDUAN FRANZ [DE] ET AL) 25 April 2000 (2000-04-25) * column 1 - column 3; claims; figures * ----- | 1-16 | |
| | | | **TECHNICAL FIELDS SEARCHED (IPC)** |

The present search report has been drawn up for all claims

| Place of search | Date of completion of the search | Examiner |
|---|---|---|
| Munich | 20 June 2012 | Gonçalves Mauger, M |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 12 00 0479

This annex lists the patent family members relating to the patent documents cited in the above-mentioned European search report.
The members are as contained in the European Patent Office EDP file on
The European Patent Office is in no way liable for these particulars which are merely given for the purpose of information.

20-06-2012

| Patent document cited in search report | | Publication date | Patent family member(s) | | Publication date |
|---|---|---|---|---|---|
| US 2010227348 | A1 | 09-09-2010 | EP | 2022859 A1 | 11-02-2009 |
| | | | EP | 2183384 A1 | 12-05-2010 |
| | | | US | 2010227348 A1 | 09-09-2010 |
| | | | WO | 2009015870 A1 | 05-02-2009 |
| EP 0293732 | A2 | 07-12-1988 | AT | 108489 T | 15-07-1994 |
| | | | AU | 617108 B2 | 21-11-1991 |
| | | | AU | 1696888 A | 08-12-1988 |
| | | | CA | 1318228 C | 25-05-1993 |
| | | | DE | 3850615 D1 | 18-08-1994 |
| | | | EP | 0293732 A2 | 07-12-1988 |
| | | | JP | 63315934 A | 23-12-1988 |
| | | | US | 5037738 A | 06-08-1991 |
| WO 2009015870 | A1 | 05-02-2009 | EP | 2022859 A1 | 11-02-2009 |
| | | | EP | 2183384 A1 | 12-05-2010 |
| | | | US | 2010227348 A1 | 09-09-2010 |
| | | | WO | 2009015870 A1 | 05-02-2009 |
| WO 2010105850 | A2 | 23-09-2010 | CA | 2755361 A1 | 23-09-2010 |
| | | | CN | 102348808 A | 08-02-2012 |
| | | | EP | 2408931 A2 | 25-01-2012 |
| | | | KR | 20110127713 A | 25-11-2011 |
| | | | US | 2012045843 A1 | 23-02-2012 |
| | | | WO | 2010105850 A2 | 23-09-2010 |
| US 7857760 | B2 | 28-12-2010 | EP | 1893084 A2 | 05-03-2008 |
| | | | US | 2006142651 A1 | 29-06-2006 |
| | | | US | 2006155180 A1 | 13-07-2006 |
| | | | US | 2006183984 A1 | 17-08-2006 |
| | | | US | 2006183985 A1 | 17-08-2006 |
| | | | US | 2006200020 A1 | 07-09-2006 |
| | | | US | 2006200970 A1 | 14-09-2006 |
| | | | US | 2006235285 A1 | 19-10-2006 |
| | | | US | 2007038044 A1 | 15-02-2007 |
| | | | US | 2007059196 A1 | 15-03-2007 |
| | | | US | 2007163880 A1 | 19-07-2007 |
| | | | US | 2010081908 A1 | 01-04-2010 |
| | | | WO | 2007002189 A2 | 04-01-2007 |
| US 6055060 | A | 25-04-2000 | AU | 702209 B2 | 18-02-1999 |
| | | | AU | 2855797 A | 29-01-1998 |
| | | | CA | 2210559 A1 | 16-01-1998 |
| | | | CN | 1174996 A | 04-03-1998 |
| | | | EP | 0819943 A2 | 21-01-1998 |
| | | | IL | 121279 A | 20-05-2001 |

EPO FORM P0459

For more details about this annex : see Official Journal of the European Patent Office, No. 12/82

## ANNEX TO THE EUROPEAN SEARCH REPORT
## ON EUROPEAN PATENT APPLICATION NO.

EP 12 00 0479

This annex lists the patent family members relating to the patent documents cited in  the above-mentioned European search report.
The members are as contained in the European Patent Office EDP file on
The European Patent Office is in no way liable for these particulars which are merely given for the purpose of information.

20-06-2012

| Patent document cited in search report | Publication date | Patent family member(s) | | Publication date |
|---|---|---|---|---|
| | | JP | 3455654 B2 | 14-10-2003 |
| | | JP | 10073535 A | 17-03-1998 |
| | | US | 6055060 A | 25-04-2000 |

EPO FORM P0459

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 0293732 A2
- US 20050214891 A1
- US 20060003397 A1
- WO 9400602 A
- EP 2022859 A
- US 5485530 A
- EP 0561653 A1
- WO 2007012494 A
- US 20070026476 A
- US 3802842 A
- US 4061468 A
- US 20060003397 A

**Non-patent literature cited in the description**

- **J. R. LAKOVICZ.** Principles of Fluorescence Spectroscopy. Springer Science and Business Media, 2006
- **T. G. SCOTT et al.** Emission Properties of NADH. Studies of Fluorescence Lifetimes... *J. Am. Chem. Soc.,* 1970, 687-695
- **J. T. SLAMA.** *Biochemistry,* 1989, vol. 27, 183
- *Biochemistry,* 1989, vol. 28, 7688